# EUROPEAN PATENT APPLICATION

(11) **EP 3 553 767 A1**
(43) Date of publication of application: **16.10.2019**
(21) Application number: 19168916.5
(22) Date of filing: 12.04.2019
(51) Int. Cl.: G09B 23/28

(54) **SIMULATED BREATHING APPARATUS**

(30) Priority: 13.04.2018 GB 201806123
(71) Applicant: Astech Projects Ltd, Runcorn, Cheshire WA7 1TQ (GB)
(72) Inventor: Berry, Andrew John, Runcorn, Cheshire WA7 1TQ (GB); Bridge, Michael James, Runcorn, Cheshire WA7 1TQ (GB)
(74) Representative: Hutchinson, Thomas Owen

(57) **Abstract**

An apparatus (100) that simulates breathing, which is suitable for use when testing inhalers (14), comprises an inlet (22) to which, in use, an inhaler (14) can be connected, and a vacuum pumping system, which has a vacuum pump (106), a high-speed valve (102) and a flow sensing means (137, 112, 104, 114) interposed between the inlet (22) and the vacuum pump (106), and a controller (126). A user interface (134) enables a user to input a target flow profile (148, 150), which may correspond to human inspiration, such that, in operation, the controller (126) can control the operation of the vacuum pump (106) and the high-speed valve (102) so that the pressure and/or flow rate within the apparatus (100) matches the pressure (150) and/or flow rate (148) of the target flow profile in real-time or near-time.

## Description

This invention relates to a simulated breathing apparatus and method a method of using the same, and in particular, a simulated breathing apparatus and method which can be used for testing medical inhalers and the like.

Many types of medicines can be administered using an inhaler, which is a device that produces a vapour or powder dispersion upon actuation, which is inhaled by a user. Pulmonary administration of drugs is the preferred option for many types of medicament, especially asthma drugs, or other drugs, which need to be taken-up by the body quickly.

The efficacy of inhalers needs to be tested as part of the regulatory processes relating to the medicament itself as well as to the "medical device", that is to say the inhaler. Even if the medicament itself is clinically effective, if the administration of the medicament is defective or inconsistent, then this can reduce the efficacy of the medicament in actual use. Therefore, not only does the efficacy of the medicament need to be tested, but also the efficacy of the administration device (inhaler).

In order to be able to assess the efficacy of an inhaler type device, it is customary practice to simulate the drug administration procedure in laboratory conditions, using a breathing simulator. The use of a breathing simulator enables tests to be carried out reproducibly and reliably under controlled conditions.

A known breathing simulator comprises an inlet aperture to which the inhaler is sealingly affixed and a vacuum source, which can be actuated on-command to simulate a user taking the drug. The inhaler can be actuated automatically during the simulated inhalation process to simulate actual use conditions and the medicament can be captured in, for example, a cascade impactor type device - to assess the delivery of the medicament under simulated conditions.

An impactor-type device is largely outside the scope of this disclosure, but for the sake of completeness, it typically comprises a number of fenestrated "stages", each being a filter-type device having apertures or perforations therein which, effectively, "sieve" the inhaled powder/vapour stream from the airflow to different degrees and simulate the interaction between the medicament and the various tissues of the patient's body (e.g. the lung structures).

After the test has been completed, the impactor can be disassembled, and the medicament recovered from each of the recovery stages yielding data relating to the effectiveness of the delivery of the medicament under those simulated conditions.

As previously stated, the function and operation of an impactor is largely outside the scope of this disclosure, and it will be appreciated that various types of impactor or other similar collection devices could be used in conjunction with a simulated breathing apparatus.

It is important that the breathing simulator mirrors, as closely as possible, the physiology of a actual person using the device. As such, the vacuum pressure, flow profile, and volume of air inhaled needs to mirror, as closely as possible, those found in a real patient during inhalation.

It has been found, empirically, that positive displacement pumping devices are generally the most effective at simulating the breathing of a patient because, in essence, human inspiration is achieved by a positive displacement process. Specifically, the rib cage expands, and the diaphragm flattens-thereby increasing the volume within the thorax, which in turn, draws air into the respiratory system.

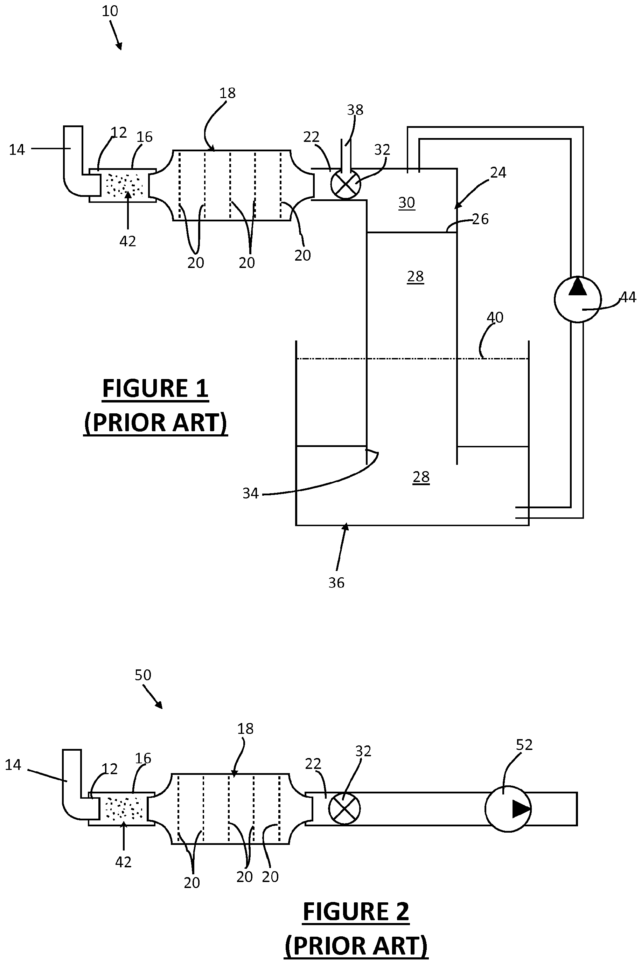

This can be simulated in known breathing simulators by a column of water, which can be released on-command to draw air in above the water level as it drops. A known breathing simulator is shown, schematically, in Figure 1 of the drawings.

Referring to Figure 1, a known breathing simulator 10 is shown, which has an inlet/mouthpiece 12 formed as an open-ended tube to which an inhaler 14 is sealingly fitted. The inlet tube 16 leads to an impactor 18, which has a number of stages 20 formed as a set of meshes or grids, which capture different sizes of particulate or vapour medicament as it flows through the device 10. At the outlet of the impactor 18, a vacuum tube 22 is sealingly connected, which leads to a vacuum column 24.

The column 24 has a hollow interior, which is filled up to a certain fill level 26 with water 28 or some other suitable liquid, which is retained in the column 24 by a vacuum 30 above the fill level 26 of the liquid. A valve 32 ensures that the vacuum 30 is preserved until actuation of the device. The lower end of the column 34 is located in a partially-filled tank 36, which forms a bariatric leg with the air pressure acting on the water 28 in the tank 36 balancing the air pressure 30 in the column 24 above the fill level 26.

When it is desired to carry out a test, the valve 32 is opened, which permits air into the column 24 via an air inlet tube located above the fill level 26. This releases the vacuum 30 above the fill level 26 of the liquid 28 and so the water column can now drop to until it reaches a new fill level 40, which equalises with a new, higher fill level 40 within the tank 36.

This process causes a volume air equivalent to the volume of the column 24 between the upper fill level 26 and the lower fill level 40 to be drawn in - via the inlet 12 of the device 10.

During the induction process, the inhaler 14 can be actuated, and this causes a cloud of particulate or vapour medicament 42 to be drawn into the system 10, where that cloud 42 is captured by the various stages 20 of the impactor 18.

After the system 10 has come to rest, the impactor 18 can be removed and disassembled: the medicament captured at each of the stages can then be recovered for later analysis. The system can be reset by re-closing the valve 32 and pumping 44 liquid 28 from the tank 36 back into the column 24, to re-fill the column 24 up to the upper fill level 26 again.

It will be appreciated, by the skilled reader, that various parameters of this known system can be user-defined, such as the flow rate (by changing the extent of opening of the valve 32), as well as the volumetric displacement (by varying the upper fill level 26). The main drawback of known systems of this type is that a continuous vacuum cannot be drawn because the volumetric displacement is limited by the volume of the column 24 between the upper 26 and lower fill levels 40, and because once started, the vacuum drawn by the column arrangement is somewhat invariable. Furthermore, it is not possible to apply a continuous vacuum using a system of this type because it is a positive displacement-type pumping system, which is effectively a single-shot system.

An alternative type of known breathing simulator is shown, schematically, in Figure 2 of the drawings, in which identical reference signs have been used to identify identical features for the sake of brevity and cross-referencing. It will be noted that the main difference between the system 50 shown in Figure 2 and that 10 shown in Figure 1 is in relation to the manner in which the vacuum is obtained.

In the device shown in Figure 2, rather than using a column to simulate inhalation of a patient, a vacuum pump 52 is used instead. The vacuum pump 52 can be controlled, for example by a computer, to control its speed, and thereby the flow rate of air through the system 50. However, even though the system shown in Figure 2 permits a continuous vacuum to be drawn, the starting and stopping characteristics of this type of system, i.e. when the pump 52 is first switched on or off, have been found to be largely unrepresentative of actual breathing conditions.

This can be ameliorated to a certain degree by controlling the valve 32 also, but if the pump 52 is switched on with the valve 32 closed, upon first opening of the valve 32, there tends to be an unrealistically large flow rate initially due to the build-up of vacuum downstream of the valve 32.

In other words, the known type of breathing simulator shown in Figure 1 has been found to be the most "realistic", albeit with certain drawbacks as outlined above; whereas the known breathing simulator shown in Figure 2 is often considered more versatile, albeit at the expense of reduced "realism". In addition, due to the internal volumes of the conduit of these known systems, there can be somewhat of a lag in the operation of these devices due to the viscoelastic nature of air. This can give rise timing and control issues, and therefore variations in the procedure from test to test.

A need therefore exists for an improved and/or alternative type of breathing simulator, which addresses the shortcomings of known breathing simulators and/or which addresses one or more of the problems outline above.

Various aspects in the invention are set forth in the appended independent claims. Preferred and/or optional features of the invention are set forth in the appended dependent claims.

According a first aspect of the invention, there is provided a simulated breathing apparatus comprising an inlet and a vacuum pumping system, the vacuum pumping system comprising a vacuum pump and a high-speed valve interposed between the inlet and the vacuum pump, a flow sensing means interposed between the valve and the pump and a controller for controlling the operation of the vacuum pump and the high-speed valve.

A second aspect of the invention provides a simulated breathing apparatus comprising: an inlet and a vacuum pumping system, the vacuum pumping system comprising: a vacuum pump; a high-speed valve interposed between the inlet and the vacuum pump; a flow sensing means interposed between the inlet and the pump; and a controller for controlling the operation of the vacuum pump and the high-speed valve, which comprises a user interface, via which a user can input a target flow profile , the target flow profile being a time-dependent flow profile, that is to say a target pressure and/or flow rate as a function of time, wherein the controller is configured to control the operation of the vacuum pump and the high-speed valve so that the actual pressure and/or flow rate within the apparatus is matched to the target flow profile in real-time or near-time.

Suitably, the controller is operatively connected to the vacuum pump, the flow monitoring device and the high-speed valve. The controller can thus be adapted to monitor the flow rate within the device using the flow monitoring device and to control the flow within the apparatus by controlling the operation of the vacuum pump and the high-speed valve. The controller can be adapted to control the operation of the high-speed valve by switching it on or off; by adjusting or controlling it step-wise, i.e. incrementally; by adjusting or controlling it infinitesimally; and/or by adjusting or controlling it continuously.

Suitably, the controller comprises a user interface, via which a user can input a target flow profile. The controller is suitably configured to control the vacuum pump and the high-speed valve (by any of the means listed above) in response to one or more outputs of the flow sensing means - so as to create a flow within the apparatus that matches the user-inputted target flow profile.

Suitably, the flow sensing means comprises a restriction interposed between the high-speed valve and the vacuum pump and a pressure sensor located upstream and a pressure sensor located downstream of the restriction. The upstream and downstream pressure sensors suitably enable the pressure within the apparatus to be monitored. In addition, the flow within the system can be monitored by monitoring a pressure differential between the upstream and downstream pressure sensors.

Suitably, the target flow profile is a time-dependent flow profile, that is to say whereby a user inputs a target pressure as a function of time and/or a target flow rate as a function of time.

The apparatus, by virtue of its controller, can control the operation of the vacuum pump and the high-speed valve so that the actual pressure and/or flow rate within the apparatus is matched to the target flow profile in real-time or near-time as the procedure proceeds.

The controller is suitably adapted to measure the pressure and/or flow rate at a relatively high sampling rate and so control the system, almost instantaneously, so that the actual flow regime within the apparatus matches the target flow profile as precisely as possible.

The sampling rate could be anything from 1Hz to 1MHz with corresponding control inputs being provided at the same, or at a different, frequency. By employing a high sampling and control rate, the actual flow parameters within the apparatus can be controlled almost instantaneously and can be made to very accurately match the target flow profile.

Preferably, the target flow profile corresponds to a human inspiration process.

The high-speed valve is preferably actuated via a high-speed actuator, such as a stepper or servo motor, which enables the opening and closing of the valve to be accurately controlled at high speed.

One advantage of using the aforedescribed system is that the high-speed valve can be set to a "closed" position, which is not necessarily actual/complete closure of the high-speed valve, but rather to a position where the flow rate within the apparatus drops to zero or substantially zero.

The benefit of this is that rather than having the valve "locked down" when in a notional closed position, it can be set slightly open, or just closed. The advantage of this is that the torque required to re-open the valve is minimal (as there is no need to overcome friction or clamping pressures first), thus reducing any opening lag, which would otherwise be present. The invention also reduces errors due to backlash in an over-tightened system.

Preferred embodiments of the invention shall now be described, by way of example only, with reference to the accompanying drawings in which:
Figure 1 is a schematic system diagram of a known breathing simulator utilising a water column;
Figure 2 is a schematic diagram of a known breathing simulator using a vacuum pump; and
Figure 3 is a schematic diagram of an embodiment of a breathing simulator in accordance with the invention.

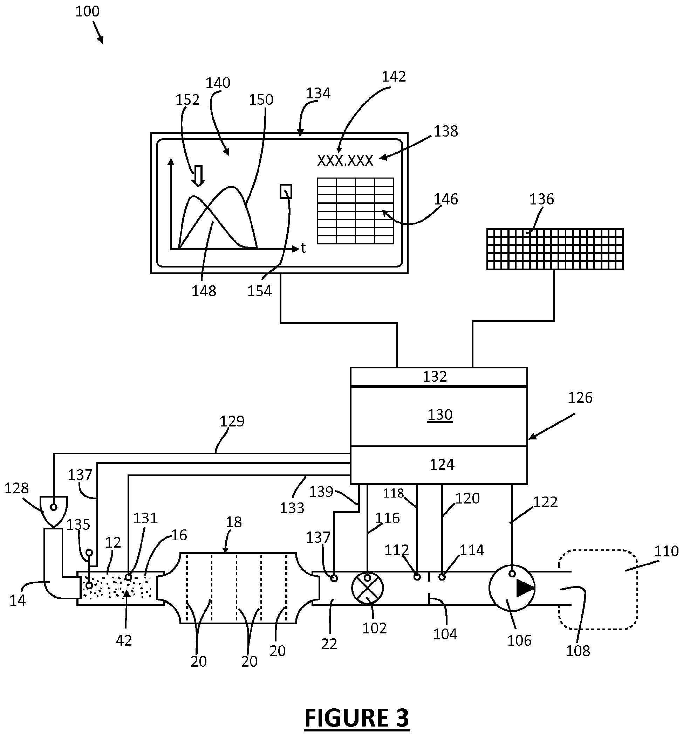

Referring now to Figure 3 of the drawings, an embodiment of a breathing simulator 100 in accordance with the invention comprises an inlet 12 to which an inhaler 14 is sealingly fitted. The inlet 12 leads to a tube 16, which connects in turn to an analyser, which in the illustrated embodiment, is an impactor 18, which comprises a number of stages 20. In other embodiments (not shown), the analyser is any piece of apparatus or equipment that could be used for dose collection and/or waste dose collection. In other embodiments, the "analyser" may simply be a tube for the purposes of calculating air flow resistance.

The outlet of the analyser is connected to a vacuum tube 22, which has a high-speed valve 102, a restriction 104 (e.g. an orifice plate) and a vacuum pump 106. An outlet 108 of the system 100 is optionally connected to an outlet filter system 110, but this is not essential.

In certain embodiments of the invention, the high-speed valve 102 and the restriction 104 are integrally formed, that is to say, with the restriction being adjustable, for example, using a servo. By placing pressure sensors 112, 114 upstream and downstream (respectively) of the adjustable restriction, the functions of the high-speed valve 102 and the restriction 104 can be combined into a single unit, but this is optional.

An upstream pressure sensor 112 is located within the tube 22 upstream of the restriction 104; and a downstream pressure sensor 114 is located in the tube 22 downstream of the restriction 104. The restriction, in the illustrated embodiment, comprises a simple orifice plate, but it could be of a more complex design, such as a venturi, if required. Control lines 116, 118, 120, 122 connect the high-speed valve 102, the upstream pressure sensor 112, the downstream pressure sensor 114 and the pump 106, respectively, to an I/OI/O bus 124 of a controller 126. An actuator 128, which actuates the inhaler 14 on-command is also provided and this too interfaces with the controller 126 via a further control line 129.

An optional temperature sensor 131 is provided at the inlet 12, which also interfaces 133 with the I/O bus 124, to measure the temperature at or near to the inflow airstream. This temperature measurement can be used to convert between mass flow and volumetric flow, as will be well-understood by the skilled reader.

The controller 126 comprises a processing unit 130, which interfaces with the I/O bus 124 to control the inhaler actuator 128, the high-speed valve 102 and the pump 106. In certain embodiments, the processor 130 also monitors the pressures obtained by the upstream 112 and the downstream 114 pressure sensors and can deduce therefrom, in addition, a flow rate within the tube 22.

A differential pressure sensor 135, which also interfaces with the I/O bus 124, measures the pressure differential between the ambient atmospheric pressure and the inlet 12 pressure. A flow meter 137, which also interfaces with the I/O bus 124 is provided in the conduit 22, upstream of the valve 102 and the orifice plate 104.

In certain embodiments, flow is measured using the aforedescribed flow meter 137. In other embodiments, flow is measured using a combination of the flow meter 137, and the upstream 112 and the downstream 114 pressure sensor measurements.

The controller 126 can be controlled by a user (not shown) via a HID interface 132, which connects to a display unit 134 and a human input device 136, 138, which, in the illustrated embodiment, is a keyboard and a touch-screen, respectively.

The display system 134 comprises a touch-screen monitor which displays a target flow profile area 140, a numerical display area 142 and a chart display area 146.

A user can input a target flow profile using the human interface devices 138, 136 to set a target flow rate 148 and/or a target pressure 150 as a function of time (t). Using the human interface devices 136, 138, a user can also set a trigger point 152, that is to say a point in time (t) at which the inhaler actuator 128 is actuated to trigger firing of the inhaler at a certain point in the cycle.

The display unit 134 also has a "start" button 154, which when pressed by a user, initiates the test procedure.

In use, the system 100 is set up as shown below with a clean analyser (impactor) 18 and an inhaler 14 affixed to the inlet 12 of the tube 16. The high-speed valve 102 is initially set at a "closed" position, which is a position where the measured flow within the tube 22 is 0 with the pump 106 switched on. The high-speed valve 102 is simply closed to a point at which the flow within the tube is measured to be 0, or substantially 0, which need not necessarily be a point at which the high-speed valve 102 is completely closed or tightened-down.

The test then proceeds.

The pump 106 and the high-speed valve 102 are controlled by the processor 130 of the controller 126 to ensure that the pressures and flow - as measured by pressure sensors 112 and 114 and/or the flow meter 137, match the inputted flow 148 and pressure 150 profile as inputted by the user via the input device 134. After the predetermined period of time, as set by the trigger point 152, the processor 130 sends a signal, via the I/O bus 124 and control line 129 to actuate the inhaler actuator 128, which causes the inhaler 14 to release a cloud 42 of particulate and/or vapour medicament into the inlet 16/12 of the system 100. The vacuum drawn by the vacuum pump causes the cloud 42 to flow into the analyser (impactor) 18, where it is collected to different degrees by the various stages 20 thereof. During the procedure, the pressure and flow within the tube 22 is continuously monitored by the flow sensor 137 and/or the pressure sensors 112, 114 and the I/O bus 124. The processor 130 calculates the optimum pump 106 and high-speed valve 102 settings to keep the pressure and flow, as a function of time, in accordance with the target flow 148 and pressure 150 as inputted by the user.

At the end of the test, the pump 106 can either be switched off and/or the high-speed valve closed in accordance with the target flow profile as set by the user of the system.

Once there is no flow in the tube 22, the analyser (impactor) 18 can be removed and disassembled so that the medicament captured by the analyser, or stages 20 of the impactor20 thereof can be analysed as necessary.

The sampling rate of the I/O bus 124 is typically high-speed sampling so that the actual pressure and flow rates within the tube 22 match, as closely as possible, the target flow rate 148 and pressure 150 in the time domain as specified by the user of the system 100.

The invention is not restricted to the details of the foregoing embodiment, which are merely exemplary of the invention.

## Claims

1. A simulated breathing apparatus (100) comprising: an inlet (22) and a vacuum pumping system, the vacuum pumping system comprising:
a vacuum pump (106);
a high-speed valve (102) interposed between the inlet (22) and the vacuum pump (106);
a flow sensing means (112, 104, 114) interposed between the inlet (22) and the pump (106); and
a controller (126) for controlling the operation of the vacuum pump (106) and the high-speed valve (22), which comprises a user interface (134), via which a user can input a target flow profile (148, 150), the target flow profile being a time-dependent flow profile, that is to say a target pressure (150) and/or flow rate (148) as a function of time, wherein
the controller (126) is configured to control the operation of the vacuum pump (106) and the high-speed valve (102) so that the actual pressure and/or flow rate within the apparatus (100) is matched to the target flow profile (148, 150) in real-time or near-time.

2. The simulated breathing apparatus (100) of claim 1, wherein controller (126) is configured to control the vacuum pump (106) and the high-speed valve (102) in response to one or more outputs of the flow sensing means (112, 114) so as to create a flow within the apparatus (100) that matches the user-inputted target flow profile (148, 150).

3. The simulated breathing apparatus (100) of claim 1 or claim 2, wherein the controller (126) is operatively connected to the vacuum pump (106), the flow monitoring device (112, 104, 114) and the high-speed valve (102), and wherein the controller (126) is adapted, in use, to:
monitor the pressure (137) and/or flow rate (112, 114) within the device using the flow sensing means; and
control the flow within the apparatus by controlling the operation of the vacuum pump (106) and the high-speed valve (102).

4. The simulated breathing apparatus (100) of any preceding claim, wherein the flow sensing means comprises a differential pressure sensor, which measures the pressure differential between any one or more of a group comprising: atmosphere (135) and the inlet (137); opposite sides (137, 112) of the high-speed valve (102); and opposite sides (112, 114) of a restriction (104).

5. The simulated breathing apparatus (100) of any preceding claim, further comprising a temperature sensor (131), whose output is used by the controller (126) to convert between mass flow and volumetric flow measurement.

6. The simulated breathing apparatus (100) of any preceding claim, wherein the flow sensing means comprises a restriction (104) interposed between the high-speed valve (102) and the vacuum pump (106) and a pressure sensor located upstream of the restriction (112) and a pressure sensor located downstream of the restriction (114), whereby a pressure within the apparatus to be monitored using either or both of the upstream (112) and downstream (114) pressure sensors, and whereby a flow rate within the system can be monitored by monitoring a pressure differential between the upstream (112) and downstream (114) pressure sensors.

7. The simulated breathing apparatus (100) of any preceding claim, wherein the flow sensing means comprises an impeller, and means for detecting a speed of rotation of the impeller in response to a flow of fluid.

8. The simulated breathing apparatus (100) of claim 6 or claim 7, wherein the restriction (104) comprises any one or more of the group comprising: an orifice plate; a Venturi; and an adjustable orifice plate of the high-speed valve (102).

9. The simulated breathing apparatus (100) of any preceding claim, wherein controller (126) is configured to measure the pressure and/or flow rate at any one or more of the group comprising: between 1Hz to 1MHz; and between 1Hz to 1MHz.

10. The simulated breathing apparatus (100) of any preceding claim, wherein the target flow profile (148, 150) corresponds to a human inspiration process.

11. The simulated breathing apparatus (100) of any preceding claim, wherein the high-speed valve (102) is actuated via a high-speed actuator.

12. The simulated breathing apparatus (100) of any preceding claim, wherein the controller (126) is configured to determine a closed position of the high-speed valve (102), which corresponds to a valve position where the flow rate within the apparatus is zero or substantially zero.

13. The simulated breathing apparatus (100) of any preceding claim, further comprising an actuator (128) for automatically actuating an inhaler (14) connected, in use, to the inlet (22) of the apparatus (100), and wherein the controller (126) is adapted to detect the firing of the inhaler (14) by the detection of a spike (peak and/or trough) in a flow measurement.

14. The simulated breathing apparatus (100) of any preceding claim, further comprising a display (140) and a human input device (136) operatively connected to the controller (126) for controlling and monitoring the operation of the apparatus (100).

15. The simulated breathing apparatus (100) of any preceding claim, wherein the controller (126) is adapted to control the pump (106) and high-speed valve (102) to produce a continuous flow, which can be used to obtain data over a period of time that is greater than 10 seconds.
